# FASCICULE DE BREVET EUROPEEN

(11) **EP 0 555 116 B1**
(45) Date de publication et mention de la délivrance du brevet: **14.04.1999**
(21) Numéro de dépôt: 93400146.2
(22) Date de dépôt: 22.01.1993
(51) Int. Cl.: A01N 47/44, A01N 39/00, A61L 15/16, A61L 31/00, A61K 31/155

(54) **Compositions à base d'antiseptiques et leurs applications**
Mittel auf Basis von Antiseptiken und ihre Verwendungen
Compositions based on antiseptics and their applications

(30) Priorité: 22.01.1992 FR 9200677; 21.05.1992 FR 9206194
(43) Date de publication de la demande: 11.08.1993
(73) Titulaire: HUTCHINSON, 75008 Paris (FR)
(72) Inventeur: Busnel, René Guy, f-91570 Bievres (FR)
(74) Mandataire: Ores, Irène

(56) Documents cités:
- EP-A- 0 099 209
- EP-A- 0 247 251
- EP-A- 0 306 389
- EP-A- 0 371 850
- EP-A- 0 407 257
- WO-A-86/02090
- WO-A-87/00005
- DE-A- 3 331 573
- DE-A- 3 443 232
- GB-A- 821 113
- GB-A- 2 132 087
- US-A- 5 013 544
- CHEMICAL ABSTRACTS, vol. 104, no. 16, 21 avril 1986, Columbus, OH (US); F. YANO, p. 393, AN 136118t

## Description

La présente invention est relative à des compositions à usage externe à base d'antiseptiques, ainsi qu'à leurs applications notamment en hygiène générale et plus particulièrement sous forme, soit de microcapsules ou de microsphérules, notamment en association avec un dispositif prophylactique en matériau élastomère, comme un préservatif, un doigtier, un gant ou analogue, un pansement, une bande, soit de mousses ou de bains.

De nombreuses compositions antiseptiques ont été décrites dans l'Art antérieur ; elles exercent, de manière générale, plutôt une activité bactériostatique ou bactéricide ; la Demande de Brevet Européen n° 0 099 209 décrit des compositions qui comprennent un alkanol en C₁ à C₄ (60 à 80 %), un agent anti-microbien du type biguanide et notamment de la chlorhexidine, et un ammonium quaternaire, qui présentent une activité biocide résiduelle ; de telles compositions de l'Art antérieur ne permettent pas l'obtention d'une protection très rapide à la fois vis-à-vis des bactéries, des virus et des champignons. Or, pour la mise en place d'un protocole de nettoyage facile à mettre en oeuvre, notamment à l'hôpital, il était important de disposer d'une composition antiseptique à action polyvalente vis-à-vis de la plupart des organismes pathogènes et éventuellement des spermatozoïdes ; de plus, dans le cadre des soins, le besoin d'une protection de l'équipe soignante, pour éviter les risques de contamination que représentent par exemple la coupure, la piqûre ou la déchirure d'un gant, nécessitait la mise au point de compositions actives dans des temps très courts (entre quelques fractions de seconde et quelques secondes, aussi bien vis-à-vis des bactéries rencontrées notamment à l'hôpital que vis-à-vis d'affections virales telles que le SIDA, les hépatites ou l'herpès), pouvant être incorporées dans les gants de protection, c'est-à-dire compatibles avec les matériaux en élastomères, ce qui n'est pas le cas des compositions de l'Art antérieur, qui aux doses efficaces utilisées, présentent l'inconvénient majeur d'être incompatibles avec les matériaux en élastomère.

La présente invention s'est en conséquence donné pour but de pourvoir à des compositions antiseptiques qui répondent mieux aux nécessités de la pratique que les compositions précédemment proposées conformément à l'Art antérieur, notamment en ce qu'elles peuvent présenter une activité polyvalente virucide, bactéricide et fongicide, assurant ainsi à l'utilisateur une protection accrue extrêmement rapide aussi bien vis-à-vis des bactéries pathogènes habituellement rencontrées chez l'Homme que vis-à-vis des virus, et plus spécifiquement HIV1, HSV et HBV, responsables respectivement du sida, de l'herpès et des hépatites.

La présente invention a pour objet des compositions à base d'antiseptiques, caractérisées en ce qu'elles sont constituées par une paire ammonium quaternaire-digluconate de chlorhexidine, dans laquelle chacun desdits antiseptiques est présent à des concentrations efficaces minimales diminuées d'au moins un facteur cinq par rapport aux concentrations efficaces minimales de chacun desdits antiseptiques pris isolément, en ce que ladite paire d'antiseptiques exerce une action rapide et polyvalente vis-à-vis des organismes suivants : bactéries à Gram positif, bactéries à Gram négatif, virus, champignons et spermatozoïdes, en ce que l'ammonium quaternaire est du chlorure de didécyldiméthylammonium, en ce que la concentration en chlorure de didécyldiméthylammonium est comprise entre 0,00005 et 0,4 % et la concentration en digluconate de chlorhexidine est comprise entre 0,00005 et 0,5 % et en ce que lesdites compositions sont susceptibles d'être incorporées dans des microsphérules ou microcapsules pouvant être incluses dans un matériau tel qu'un matériau en élastomère, lesquelles compositions n'étant libérées que lors d'une coupure, d'une piqûre ou d'une déchirure dudit matériau.

De telles compositions ont l'avantage de permettre l'utilisation des antiseptiques la comprenant à des concentrations inférieures à celles auxquelles ils exercent leur action antiseptique lorsqu'ils sont utilisés seuls.

En effet ces concentrations représentent une fourchette pour laquelle les compositions conformes à l'invention exercent leur action polyvalente contre les différents organismes pathogènes : virus, bactéries, champignons et les spermatozoïdes.

Au sens de la présente invention, polyvalence signifie à la fois polyvalence intergroupe (c'est-à-dire activité à la fois vis-à-vis des organismes cités) et polyvalence intragroupe (c'est-à-dire activité à la fois vis-à-vis des bactéries à Gram + et à Gram -, de différents virus tels que HIV-1, HSV, HBV et de différents champignons).

Selon un mode de réalisation avantageux desdites compositions, elles sont constituées par une paire ammonium quaternaire-digluconate de chlorhexidine dans laquelle les rapports chlorure de didécyldiméthylammonium:digluconate de chlorhexidine sont compris entre 99,5:0,5 et 84:16.

De manière avantageuse, lesdites compositions comprennent ces deux antiseptiques à des concentrations identiques, de préférence entre 0,00005 et 0,04 %.

Egalement de manière avantageuse, lesdites compositions sont associées à au moins un agent de dissolution choisi dans le groupe constitué par l'eau et les polyols.

Selon une disposition avantageuse de ce mode de réalisation, lesdits polyols sont choisis parmi le polypropylèneglycol, le polyéthylèneglycol ou le glycérol.

De manière surprenante, de telles compositions présentent une stabilité nettement améliorée, quelle que soit la concentration en antiseptiques (chlorure de didécyldiméthylammonium et digluconate de chlorhexidine), lorsqu'ils sont présents dans les proportions précisées ci-dessus (99,5:0,5 et 84:16) et trouvent ainsi application dans une formulation qui pourra être introduite dans des microcapsules ou qui pourra être directement utilisée (mousses, bains à usage local, nettoyage instrumental).

De manière surprenante, les compositions conformes à l'invention présentent une activité antiseptique polyvalente rapide (quelques fractions de seconde à quelques secondes) vis-à-vis des organismes pathogènes : action bactéricide vis-à-vis des bactéries à Gram positif et à Gram négatif, action fongicide (notamment flores opératoire et vaginale) et action virucide vis-à-vis de HIV1, HSV et HBV très rapide, et éventuellement action spermicide, à des doses pour lesquelles ses constituants ne présentent pas lesdites activités. De telles compositions présentent donc une synergie d'action particulièrement intéressante.

Les compositions conformes à l'invention, outre leur synergie d'action vis-à-vis des différents organismes précités, sont particulièrement bien adaptées à leur emploi sous forme de microcapsules (présence d'un seul ammonium quaternaire, concentrations faibles permettant de rester en phase liquide, sans problème de formation ultérieure de précipité).

Ces compositions antiseptiques conformes à l'invention, incorporées dans des microcapsules, conservent néanmoins leurs propriétés bactéricides, fongicides, virucides ou spermicides, et peuvent être également incorporées dans un matériau en élastomère à usage de préservatif, de doigtier, de gant ou analogue ou bien dans une bande de tissu ou un pansement.

Ces compositions, incluses dans des microcapsules et incorporées dans des matériaux en élastomère (gants de protection, par exemple), protègent l'utilisateur en permettant, par exemple, la désinfection instantanée d'une aiguille usagée et éventuellement contaminée lors d'une piqûre ou d'une déchirure accidentelle desdits gants chargés en microcapsules.

En outre, les compositions conformes à l'invention contenant au moins un polyol, dans les proportions définies ci-dessus, sont particulièrement bien adaptées à la préparation de microcapsules, qui nécessitent, lors de leur incorporation dans un dispositif en matériau élastomère, notamment, une étape de chauffage à des températures supérieures à 80°C ; de telles compositions présentent également l'avantage d'éviter l'évaporation de l'agent de dissolution et donc de conserver les propriétés des solutions microencapsulées ; de plus, de telles compositions permettent d'obtenir une meilleure diffusion desdits antiseptiques, au contact de fluides biologiques tels que le sang, le sperme, les sécrétions vaginales et autres.

Les compositions conformes à l'invention constituent également une protection virucide, bactéricide, fongicide et/ou spermicide efficace, lorsqu'elles sont associées à des véhicules pharmaceutiquement acceptables, pour une utilisation locale au niveau des muqueuses, (mousses, pour une utilisation vaginale, scrub, crème, suppositoires, ovules).

Les compositions conformes à l'invention constituent également un produit d'hygiène ou de désinfection à usage externe avantageux (nettoyage d'outils chirurgicaux, dentaires et gynécologiques).

En fonction des concentrations des différents constituants, on obtient soit une composition à action spécifique (virucide, bactéricide, fongicide ou spermicide), soit une composition à action polyvalente.

Dans le cas où lesdites compositions sont utilisées comme spermicide, elles sont avantageusement associées à un diluant choisi dans le groupe constitué par les protéases d'origine végétale (bromélaine) et les enzymes lytiques (trypsine).

Outre les dispositions qui précèdent, l'invention comprend encore d'autres dispositions, qui ressortiront de la description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

### EXEMPLE 1 : Action virucide des compositions conformes à l'invention vis-à-vis des virus HIV (Sida).

### I - Antiseptique cationique-sel de chlorhexidine.

### A) PROTOCOLE OPERATOIRE.

### 1. Matériel :

### a) Produits utilisés :

Les concentrations indiquées sur les tableaux correspondent aux concentrations finales en Bardac 22.60® (chlorure de didécyldiméthylammonium).

### b) Milieu utilisé pour la culture cellulaire :

L'étude de la cytotoxicité étant réalisée sur une lignée T, le milieu de culture correspond à du RPMI 1640 supplémenté par 10 % de sérum de veau foetal (BIO-LAB), 1 % de glutamine (GIBCO), 1 % d'antibiotiques (PSN, GIBCO).

Pour l'étude du pouvoir infectieux résiduel après traitement des préparations virales, le milieu de culture des lymphocytes T humains normaux (milieu complet) correspond au milieu ci-dessus auquel sont ajoutés 10 % d'interleukine II, du polybrène (2 µg/ml) et du sérum anti-interféron humain 1/2500 (M.A. REY et al., Biochem. Biophys. Res. Com., 1984, 121, 126-133).

### c) Cellules :

La cytotoxicité est étudiée sur un clone de cellules CEM, lignée de lymphocytes T prématures.

L'infectivité est étudiée sur des lymphocytes T obtenus à partir du sang périphérique d'un donneur humain sain, et séparés sur un gradient de Ficoll.

Les lymphocytes sont stimulés pendant 3 jours par de la phytohémagglutinine P (PHA-P), diluée au 1/500 et cultivés dans du milieu complet. Les cellules T blastiques sont infectées par le virus, traité ou non, pour mettre en évidence le pouvoir infectieux résiduel.

### d) Virus HIV1 :

Le virus est obtenu à partir d'un surnageant de culture de la lignée CEM infectée par HIV1. Cette lignée T lymphoblastoïde infectée par HIV1 est utilisée pour la production virale.

Le titre des surnageants utilisés est évalué après dosage de l'activité transcriptase inverse (ATI) du virus.

Dans les essais qui suivent, on a utilisé un surnageant dont l'ATI est de 1,8.10⁶ cpm/ml. 2. **Méthodes** :

### a) Etude de la cytotoxicité des composés pour des cellules T :

Cellules : suspension cellulaire du clone de CEM à 1.10⁶ cellules/ml.
Produits : différentes dilutions dans du milieu RPMI 1640 sont réalisées à partir des composés.

Sur une plaque de culture cellulaire à 24 puits, 0,5 ml de chacune des dilutions sont incubés avec 0,5 ml de suspension cellulaire du clone CEM (5.10⁵ cellules) dans un incubateur à 37°C.

La croissance cellulaire est évaluée après comptage des suspensions cellulaires au bleu trypan par rapport à un témoin de cellules non traitées.

### b) Action des produits seuls ou en association, conformément à l'invention, sur le pouvoir infectieux du virus HIV1 pour des lymphocytes T humains normaux :

- Préparation de la solution virale :
   plusieurs tubes contenant 1 ml de surnageant viral, ATI 1,8.10⁶ cmp/ml sont concentrés par ultracentrifugation. Les culots de virus ainsi obtenus sont repris soit par chacune des dilutions de produit réalisées dans de l'eau bidistillée stérile, soit par de l'eau (virus témoin non traité).
- Traitement des préparations virales :
   les préparations virales concentrées sont toutes traitées selon le protocole indiqué ci-dessous :
   . échantillons viraux traités :
      les culots de virus concentrés par ultracentrifugation sont resuspendus dans 100 µl de chacune des concentrations en produits étudiés et incubés pendant 10 minutes.
   . échantillon viral témoin :
      témoin virus non traité : le produit est remplacé par 100 µl d'eau bidistillée stérile.

   A la suite de ce temps d'incubation de 10 minutes, le virus présent dans chaque échantillon est rincé puis reconcentré par addition de 12 ml de milieu RPMI 1640 et ultracentrifugation. Les culots de virus sont alors repris dans 1 ml de milieu complet et utilisés pour l'infection de lymphocytes T humains normaux.
- Mesure de l'infectivité des échantillons :
   4.10⁶ lymphocytes T humains normaux sont infectés avec chacune des préparations virales traitées ou témoins indiquées ci-dessus. L'infection des cellules T est réalisée selon une méthode précédemment décrite (F. BARRE-SINOUSSI et al., Science, 1983, 220, 868-871) : en résumé 4.10⁶ cellules en suspension dans 1 ml de milieu complet sont incubées avec 1 ml de chacune des préparations virales traitées ou témoins pendant 1 heure à 37°C.
   Après 2 lavages par plusieurs ml de milieu de culture, les suspensions cellulaires sont ajustées à la concentration de 1.10⁶ cellules par ml. Le jour de l'infection est considéré comme jour 0.
   . Cellules témoin :
      4.10⁶ lymphocytes T sont cultivés et passés tous les 3 ou 4 jours dans les mêmes conditions que tous les autres échantillons.
      Ce témoin correspond au témoin négatif de la production virale au cours de la manipulation.
      La production virale au cours du temps est déterminée en mesurant l'activité transcriptase inverse présente dans les surnageants de culture selon le protocole décrit ci-dessous.
   . Mesure de l'activité transcriptase inverse des préparations virales témoins et des échantillons viraux traités par les produits :
      ce dosage permet d'évaluer la présence de virus résiduel non inactivé dans l'échantillon de départ. Il est réalisé à partir de 1 ml de surnageant de culture,
prélevé tous les 3 ou 4 jours et ultracentrifugé.

Les activités enzymatiques sont mesurées dans 50 µl d'un mélange réactionnel contenant 50 mM Tris pH 7,8, 20 mM MgCl₂, 20 mM KCl, 2 mM dithiotréitol, 0,25 OD/ml oligo dT, 0,25 OD/ml poly rA, 0,1 % Triton X 100 et 2,5 µCi 3H DTTP.

Après une heure d'incubation à 37°C, la réaction est arrêtée par addition de 10 µl d'EDTA 0,2 M et les échantillons sont déposés sur membranes DE81.

Après plusieurs lavages par Na₂HPO₄ 5 %, puis par l'eau distillée, les membranes sont séchées et la radioactivité est mesurée à l'aide d'un compteur à scintillation β (PACKARD).

### B) RESULTATS.

Effet d'un traitement des échantillons viraux par les différents produits sur l'infectivité du virus HIV1.

**TABLEAU I**

| | DOSAGE DE L'ATI DANS LES SURNAGEANTS DE CULTURES INFECTEES (cpm/ml) | | | | | | | | |
|---|---|---|---|---|---|---|---|---|---|
| | J3 | J6 | J10 | J13 | J17 | J20 | J23 | J27 | J30 |
| Bardac® (%) | | | | | | | | | |
| 0,01 | 334 | 222 | 1768 | 1592 | 190 | 390 | 1328 | 780 | 1000 |
| 0,006 | 320 | 326 | 204 | 248 | 364 | 180 | 328 | 588 | 196 |
| 0,004 | 346 | 298 | 160 | 170 | 350 | 218 | 444 | 244 | 264 |
| 0,002 | 464 | 2472 | 16066 | 24902 | 77754 | 75554 | 101394 | 80560 | 64692 |
| 0,0004 | 702 | 18136 | 117326 | 74214 | 50674 | 22478 | 13806 | 7800 | 4426 |

| Chlorhexidine (%) | | | | | | | | | |
|---|---|---|---|---|---|---|---|---|---|
| 0,01 | 262 | 274 | 200 | 250 | 230 | 272 | 456 | 420 | 276 |
| 0,005 | 360 | 374 | 580 | 742 | 1902 | 1126 | 1226 | 528 | 720 |
| 0,002 | 252 | 352 | 1304 | 1752 | 4288 | 6138 | 4614 | 1616 | 4528 |

| Bardac® + Chlorhexidine (%) | | | | | | | | | |
|---|---|---|---|---|---|---|---|---|---|
| 0,006 + 0,01 | 336 | 202 | 116 | 226 | 280 | 236 | 342 | 510 | 240 |
| 0,006 + 0,005 | 326 | 196 | 210 | 384 | 274 | 166 | 280 | 406 | 304 |
| 0,006 + 0,002 | 286 | 240 | 196 | 282 | 192 | 274 | 376 | 478 | 330 |
| 0,004 + 0,01 | 302 | 326 | 210 | 222 | 240 | 350 | 366 | 278 | 248 |
| 0,004 + 0,005 | 298 | 288 | 206 | 344 | 170 | 346 | 288 | 456 | 282 |
| 0,004 + 0,002 | 228 | 268 | 208 | 232 | 1200 | 274 | 254 | 422 | 250 |
| 0,002 + 0,01 | 666 | 262 | 222 | 200 | 244 | 394 | 650 | 322 | 280 |
| 0,002 + 0,005 | 364 | 246 | 314 | 308 | 4386 | 308 | 268 | 446 | 254 |
| 0,002 + 0,002 | 998 | 222 | 666 | 908 | 748 | 1206 | 1110 | 370 | 336 |
| 0,0004 + 0,01 | 2163 | 234 | 186 | 226 | 204 | 250 | 344 | 352 | 226 |
| 0,0004 + 0,005 | 438 | 246 | 824 | 1142 | 2340 | 2190 | 2120 | 1094 | 1466 |
| 0,0004 + 0,002 | 576 | 3362 | 21734 | 36246 | 39364 | 40126 | 18480 | 6530 | 10476 |
| T1 | 1516 | 3120 | 24822 | 52912 | 49884 | 30540 | 21800 | 4500 | 3464 |
| T2 | 342 | 4688 | 61384 | 64284 | 69172 | 15366 | 12364 | 3500 | 3790 |
| T3 | 1594 | 45068 | 75046 | 56928 | 12872 | 8932 | 5468 | 1050 | 1746 |
| T4 | 232 | 220 | 218 | 202 | 190 | 268 | 394 | 244 | 308 |
| T1 : Témoin virus traité par 100 µl d'eau bidistillée T2 : Témoin virus traité par 100 µl d'eau bidistillée T3 : Témoin virus contrôle T4 : Témoin cellules non infectées | | | | | | | | | |

Ce Tableau montre bien la synergie d'action de l'association Bardac®/digluconate de chlorhexidine à faible concentration (concentration 0,002 + 0,002) qui montre l'absence de virus au 30ème jour ; en effet, pour ces mêmes concentrations, les deux produits utilisés seuls ne sont pas virucides.

### EXEMPLE 2 : Action virucide des compositions conformes à l'invention pour les virus HBV.

### I - Action sur la dégradation in vitro de l'antigène du virus de l'hépatite B (AgHBs) du virus de l'hépatite B : virucide + sérum humain de malade (1/1000ème).

### A. Mélanges testés :

| Bardac® % + CHG* % | Bardac® seul | CHG seul |
|---|---|---|
| 0,2 - 0,25 | 0,2 | 0,25 |
| 0,1 - 0,1 | 0,1 | 0,1 |
| 0,04 - 0,05 | 0,04 | 0,05 |
| 0,2 - 0,05 | | |
| 0,04 - 0,25 | | |

| | | |
|---|---|---|
| * CHG correspond à une abréviation pour digluconate de chlorhexidine. | | |

### B. Résultats :

| Virucide testé | AgHBs résiduel (ratio) |
|---|---|
| Bardac® 0,2 % | 1,35 |
| Bardac® 0,1 % - CHG 0,1 % | 2,18 |
| Bardac® 0,2 % - CHG 0,05 % | 3,07 |
| Bardac® 0,2 % - CHG 0,25 % | 3,23 |

Le mélange Bardac® 0,1 % - CHG 0,1 % présente une activité intéressante alors que la dose est diminuée de l'ordre de 50 % par rapport au Bardac® 0,2 % seul.

### II - Essais in vivo chez le canard (voie I.V.).

- Bardac® 0,02 % (dose 100 fois inférieure à celle qui est efficace *in vitro* pour le Bardac® seul) - CHG 0,025 % contre un inoculum à 2.10⁷ vge/canard protège 3/3 canards (figure 2).
- Bardac® 0,1 % (dose 5 fois plus forte que dans une composition conforme à l'invention pour obtenir une protection équivalente) contre un inoculum à 2.10⁷ vge/canard (vge = viral genoma equivalent) protège 4/4 canards (figure 3).

Il en résulte qu'*in vivo*, pour des faibles concentrations de mélange comme 0,02 - 0,025 %, il y a déjà protection, ce qui confirme que la suppression de l'activité ADN polymérase est un bon marqueur d'efficacité des virucides testés.

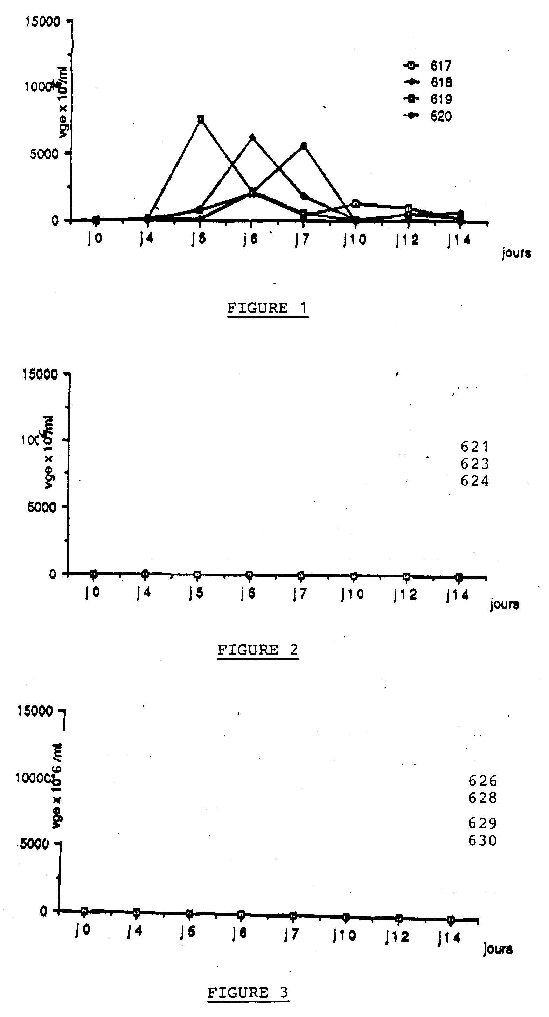

La figure 1 correspond à un témoin d'infection : 4/4 canards inoculés avec 2.10⁷ vge développent une virémie dont le pic se situe entre 5 et 10 jours.

### III - Conclusion générale sur les différents mélanges testés.

Seul l'antigène du virus de l'hépatite B résiduel permet de discriminer parmi les mélanges Bardac®-CHG testés.

Un mélange à concentrations égales de Bardac® et CHG semble optimal et s'avère *in vivo* protecteur dès la concentration 0,02 - 0,025 contre un inoculum contenant 2.10⁷ vge/canard (soit environ 3 000 DI 50 par canard).

Il apparaît que 0,02 % de Bardac® en association avec 0,025 de CHG est aussi efficace que le Bardac® à 0,1 %. Cette diminution d'un facteur 5 est significative et importante, notamment dans l'application d'une composition conforme à l'invention en association avec un matériau en élastomère, le Bardac® étant particulièrement toxique pour l'élastomère.

### EXEMPLE 3 : Action virucide des compositions conformes à l'invention contre les HSV.

### I - Essais in vitro.

### a) Tests réalisés :

### - La souche virale :

Il s'agit d'une souche d'Herpès virus *Hominis* type 1, souche Bey (HSV1), elle est entretenue sur cellules fibroblastiques humaines ou sur cellules KB et a subi un nombre de passages importants, sur l'un ou l'autre type de cellules.

On utilise un lot viral congelé à -80°C après obtention d'un effet cytopathique ayant atteint au moins 80 % des cellules, sans une centrifugation préalable, qui aurait débarrassé l'inoculum de la majorité des débris cellulaires ; en effet, il apparaît que l'infectivité du virus disparaît très rapidement lorsqu'il est extracellulaire, et, en conséquence, on est plus près de la réalité de l'infection *in vivo* lorsqu'on utilise un lot viral dans lequel des virions peuvent encore se trouver dans des débris cellulaires.

Ce lot a été titré, après répartition en aliquots de 0,5 ml, conservés à -80°C, sur cellules fibroblastiques humaines :
. par dilution de 10 en 10 et inoculations de 8 tubes de cultures par dilution : 10⁶/ml ;
. par inoculation de cultures en boîtes de Pétri (35 mm) et adjonction de sérum anti-HSV1 dans le surnageant pour neutraliser les virions qui pourraient être libérés dans le milieu liquide, permettant ainsi de compter des plages : 100 à 150 unités formant plages par boîte inoculée avec 0,5 ml d'une solution virale diluée à 10⁻⁴.

On réalise des contrôles de cytotoxicité des produits pour les cultures cellulaires utilisées dans les tests, c'est-à-dire les cellules fibroblastiques humaines.

Les cellules sont cultivées en milieu de Dulbecco enrichi en sérum de veau foetal à 10 % ; au moment de leur utilisation, les cellules sont lavées et on utilise le même milieu de culture, mais dépourvu de sérum de veau.

Pour chaque produit testé ou chaque composition, on a utilisé 10 tubes de culture cellulaire et après inoculation du produit, les tubes ont été surveillés pendant trois jours.

### b) Action observée avec les différents produits seuls :

Chaque produit a été successivement utilisé pur, et aux dilutions 10⁻¹, 10⁻², 10⁻³ et ajouté aux tubes de culture contenant 1 ml de milieu nutritif, sous le volume de 0,1 ml.

Les résultats ont été les suivants :
. Bardac®, solution à 0,04 % : non toxique à la dilution 10⁻³,
. Digluconate de chlorhexidine, solution à 0,05 % : aucune toxicité.

### c) Action virucide immédiate observée avec des mélanges conformes à l'invention :

### 1. Mesure de l'activité :

### * Protocole :

on mélange 0,2 ml de suspension virale avec 0,2 ml d'une des solutions à tester (voir Tableau ci-après) ; puis on fait une dilution immédiate de 0,2 ml de mélange à 10⁻⁴ ce qui stoppe l'activité du produit. Cette dilution du mélange est déposée, sous un volume de 1 ml sur une couche de cellules Véro cultivées en boîte de Pétri de 6 cm de diamètre.

Après une heure d'adsorption, on retire le liquide surnageant et on le remplace par 5 ml de milieu de culture sans sérum d'animal, mais auquel on a ajouté 1 % d'un sérum polyclonal de lapin anti-herpès virus type 1.

Ceci ne gêne pas la multiplication du virus intra-cellulaire mais neutralise les particules qui passent dans la phase liquide lors de la destruction des cellules infectées ; au bout de 4 à 5 jours, on observe à l'oeil nu des zones de lyse cellulaire qui s'étendent en tache d'huile et correspondent chacune théoriquement à la multiplication d'une unité formant plage" (UFP) présente dans la dilution virale qui a été déposée sur la culture. Une unité formant plage peut être grossièrement évaluée comme équivalente à une particule infectante ou encore un virion.

**TABLEAU II**

| Nombre d'unités formant plage par millilitre de virus dilué à 10⁻⁴ (titrage effectué sur 3 boîtes de culture) à J4. | | |
|---|---|---|
| Bardac® 0,02 % | Bardac® 0,02 % | Témoin virus à 10⁻⁴ 10⁻⁵ 10⁻⁶ |
| 30/125/45 | + Digluconate 0,025% 17/26/16 | I/223/51 |
| Bardac® 0,02 % | Bardac® 0,02 % | Témoin virus à 10⁻⁶ |
| 26/17/20 | + Digluconate 0,05 % 9/6/8 | 14/32/30 |

### d) Délais d'apparition de l'activité :

### 1) on mélange 0,2 ml de la suspension virale non diluée avec 0,2 ml de chacun des 2 mélanges suivants

- Bardac® 0,04 % + CHG (digluconate) 0,05 %,
- Bardac® 0,02 % + CHG 0,025 %.

Après incubation de une, 5 et 15 minutes, une goutte de mélange est inoculée dans 5 tubes de culture et surveillée pendant 5 jours.

A titre de témoin, on inocule le virus dilué de la même façon dans du milieu de culture, à la place des mélanges.

### 2) Tableau III : Résultats observés à J2 sur 5 tubes pour les deux mélanges précités :

**TABLEAU III**

| Virus + Mélange | | Virus Témoin |
|---|---|---|
| Effet cytopathique (à J2) | | |
| 2/5 | 1 minute | 5/5 |
| 0/5 | 5 minutes | 5/5 |
| 0/5 | 15 minutes | 5/5 |

### II - Essais in vivo.

Des tests effectués sur des souris Nude, soit piquées directement avec une aiguille intra-musculaire trempée dans une suspension virale, soit piquées après passage de l'aiguille infectée (c'est-à-dire trempée dans une suspension virale) à travers des microcapsules telles que décrites ci-après (Exemple 8, échantillon 1) recouvertes de latex, montrent que le passage de l'aiguille à travers les microcapsules permet une diminution instantanée des taux d'infection des animaux tout à fait significative.

Le protocole opératoire est similaire à celui de l'Exemple 8, à l'exception des milieux de culture qui doivent être adaptés au virus herpétique.

### EXEMPLE 4 : Action bactéricide des compositions conformes à l'invention.

### ESSAI 1

### I - Matériel.

### A) Microorganismes testés :

- souches de référence :
   . *Pseudomonas aeruginosa* CIP A22
   . *Escherichia coli* CIP 54127
   . *Staphylococcus aureus* CIP 53154 souche Oxford.
- souches hospitalières :
   . *Pseudomonas aeruginosa*
   . *Escherichia coli*
   . *Staphylococcus aureus*
   . *Proteus vulgaris*
   . *Candida albicans*.

### B)- Membranes millipore : HAE P047S0 (0,45 µm)

- Gélose Mueller Hinton 2 : 4 3301 (Biomérieux)
- Eau distillée stérile et apyrogène (Biosedra).

### II - Protocole.

### A) Entretien des souches :

Chaque souche bactérienne a subi trois repiquages, sur géloses Mueller Hinton, à 24 heures d'intervalle avant son utilisation.

### B) Préparation des inocula bactériens :

### - suspension bactérienne initiale : A

Préparée à partir d'un isolement bactérien, elle doit correspondre à une opacité équivalente au point 1 de McFarland. Elle sera utilisée pour les essais définitifs.

### - suspension utilisée pour les contrôles et les essais préliminaires : B

Elle est préparée par dilutions successives de la suspension A jusqu'à 10⁻⁶.

### C) Dénombrements de contrôle :

1 ml de la suspension B est ensemencé "dans la masse" d'une gélose Mueller Hinton.

### D) Dénombrements témoins de filtration :

1 ml de la suspension B est filtrée sur une membrane millipore et lavée par 50 ml d'eau distillée.

Numération des colonies après 24 heures à 37°C.

### E) Essais préliminaires :

Pour chacun des 4 mélanges antiseptiques et pour chaque souche bactérienne, l'essai suivant est réalisé :
- filtration de 1 ml du mélange antiseptique
- lavage (2 fois 50 ml d'eau distillée)
- filtration de 1 ml de la suspension B
- rinçage par 50 ml d'eau distillée
- numération des colonies sur la membrane après 24 heures à 37°C.

### F) Essais définitifs :

Chaque mélange antiseptique à double concentration est placé pendant, 1, 5 et 10 min au contact de 1 ml de la suspension A puis filtré.

Après les lavages définis par l'essai préliminaire, les membranes sont placées à 37°C pour 24 heures.

### III - Interprétation des résultats.

Dans le protocole adopté, un mélange à visée antiseptique est considéré comme bactéricide s'il réduit, après un temps de contact défini, l'inoculum bactérien d'au moins 5 logarithmes.

Cet effet est visualisé par le dénombrement, sur les membranes des essais définitifs, d'un nombre de colonies par rapport à celui observé dans l'essai préliminaire correspondant à la même souche et au même mélange.

### Résultats :

### . Efficacité à t = 1 min : E ou N.E. (efficace ou non efficace).

| | Mélange n° 1 | Mélange n° 2 |
|---|---|---|
| | Bardac® 4 % | - Bardac® 0,04 g/100 ml |
| | | - Digluconate 0,05 g/100 ml |
| *S. aureus* CIP 53154 | E | E |
| *E. coli* CIP 54127 | E | E |
| *P. aeruginosa* CPI A22 | E | E |
| *S. aureus* | E | E |
| *E. coli* | E | E |
| *P. aeruginosa* | NE | NE |
| *P. vulgaris* | NE | NE |
| *C. albicans* | E | E |

### . Efficacité à t = 5 min :

| | Mélange n° 1 | Mélange n° 2 |
|---|---|---|
| | Bardac® 4 % | - Bardac® 0,04 g/100 ml |
| | | - Digluconate 0,05 g/100 ml |
| *S. aureus* CIP 53154 | E | E |
| *E. coli* CIP 54127 | E | E |
| *P. aeruginosa* CPI A22 | E | E |
| *S. aureus* | E | E |
| *E. coli* | E | E |
| *P. aeruginosa* | NE | NE |
| *P. vulgaris* | NE | NE |
| *C. albicans* | E | E |

### . Efficacité à t = 10 min :

| | Mélange n° 1 | Mélange n° 2 |
|---|---|---|
| | Bardac® 4 % | - Bardac® 0,04 g/100 ml |
| | | - Digluconate 0,05 g/100 ml |
| *S. aureus* CIP 53154 | E | E |
| *E. coli* CIP 54127 | E | E |
| *P. aeruginosa* CPI A22 | E | E |
| *S. aureus* | E | E |
| *E. coli* | E | E |
| *P. aeruginosa* | E | E |
| *P. vulgaris* | E | E |
| *C. albicans* | E | E |

### IV - Conclusion.

- Après 1 minute de contact aucun mélange n'est efficace sur toutes les souches étudiées. Le mélange n° 2 est cependant le plus efficace puisque seule une souche de *Proteus vulgaris* semble insensible.
- Après 5 minutes de contact, le mélange n° 2 pourrait être considéré comme efficace s'il n'y avait la même souche de *Proteus vulgaris* qui résiste au produit même après 5 minutes.
- Après 10 minutes de contact, les mélanges n° 1 et 2 donnent un résultat satisfaisant sur l'ensemble des souches étudiées.

### ESSAI 2

Un autre essai a été réalisé à partir des solutions suivantes :
- 100 ml d'une solution à 0,1 % de digluconate de chlorhexidine (pH 6) et
- 100 ml d'une solution à 0,1 % de Bardac 22®.

L'activité de ces solutions, seules ou en combinaison a été plus particulièrement testée vis-à-vis de *Staphylococcus aureus* ATCC 6538, *Escherichia coli* ATCC 11229 et *Candida albicans* ATCC 10231.

Les tests ont été réalisés conformément au protocole décrit dans le Chapitre I/2.1 et 2.2 de *Richtlinien für die Prüfung und Bewertung chemischer Desinfektionsverfahren der DGHM*, Stand 1.1., 1981).

Les Tableaux A, B et C illustrent respectivement l'activité bactéricide et fongicide d'une solution aqueuse à 0,1 % de digluconate de chlorhexidine, d'une solution aqueuse à 0,1 % de Bardac 22® et d'un mélange à part égale des deux composés. Les protocoles dont ces Tableaux illustrent les résultats comprennent une incubation de 72 heures à 37°C et l'utilisation, comme témoins, des substances inactivantes suivantes :
- A :: Tween® 80 3 % + lécithine 0,3 % + cystéine 0,1 % ;
- B :: Tween® 80 3 % + saponine 3 % + cystéine 0,1 % + histidine 0,1 % ;
- C :: Tween® 80 3 % + lécithine 0,3 % + histidine 0,1 % + thiosulfate de sodium 0,5 %.

Dans ces Tableaux, le signe "+" indique une croissance et le signe "-" l'absence de croissance.

Le Tableau A suivant, qui illustre l'activité bactéricide et fongicide d'une solution aqueuse à 0,1 % de digluconate de chlorhexidine (1 % de la solution test correspond à 0,001 % de digluconate), montre l'inhibition de la croissance de *S. aureus* et de *E. coli* par une dilution à 0,5 % de la solution de digluconate de chlorhexidine précitée, correspondant à une concentration en substance active de 0,0005 % ; *C. albicans* est inhibé par une dilution à 1 % de ladite solution (0,001 % de digluconate de chlorhexidine). Dans le Tableau B, le chlorure de didécyldiméthylammonium montre un effet encore plus prononcé d'inhibition des organismes à Gram positif *S. aureus* et *C. albicans* avec une dilution à 0,1 % (= 0,0001 % de chlorure de didécyldiméthylammonium) et une inhibition de *E. coli* (organisme à Gram négatif) avec une dilution à 0,5 % (= 0,0005 % de chlorure de didécyldiméthylammonium) (Tableau B).

Le Tableau C montre que l'association de ces deux antiseptiques permet l'inhibition de la croissance des microorganismes précités à des dilutions significativement plus importantes (action vis-à-vis de *E. coli* et *C. albicans* à des concentrations de 0,00025 % de digluconate de chlorhexidine et de 0,00025 % de chlorure de didécyldiméthylammonium ; action vis-à-vis de *S. aureus* pour une concentration de 0,00005 % pour chacun des deux antiseptiques précités).

**TABLEAU A**

| (digluconate de chlorhexidine seul : 1 % de solution → 0,001 % de digluconate). | | | | | | | | | | | | |
|---|---|---|---|---|---|---|---|---|---|---|---|---|
| Essai Souche | *S. aureus* ATCC 6538 | | | | *E. coli* ATCC 11229 | | | | *Candida albicans* ATCC 1031 | | | |
| Conc. % (vol/vol) | non | A | B | C | non | A | B | C | non | A | B | C |
| 1,0 | - | + | + | + | - | + | + | + | - | + | + | + |
| 0,5 | - | + | + | + | - | + | + | + | + | + | + | + |
| 0,1 | + | + | + | + | + | + | + | + | + | + | + | + |
| 0,05 | + | + | + | + | + | + | + | + | + | + | + | + |
| 0,025 | + | + | + | + | + | + | + | + | + | + | + | + |
| 0,0125 | + | + | + | + | + | + | + | + | + | + | + | + |
| 0,00625 | + | + | + | + | + | + | + | + | + | + | + | + |

**TABLEAU B**

| (Bardac 22® seul, 1 % de solution → 0,001 % de Bardac®) | | | | | | | | | | | | |
|---|---|---|---|---|---|---|---|---|---|---|---|---|
| Essai Souche | *S. aureus* ATCC 6538 | | | | *E. coli* ATCC 11229 | | | | *Candida albicans* ATCC 1031 | | | |
| Conc. % (vol/vol) | non | A | B | C | non | A | B | C | non | A | B | C |
| 1,0 | - | + | + | + | - | + | + | + | - | + | + | + |
| 0,5 | - | + | + | + | - | + | + | + | - | + | + | + |
| 0,1 | - | + | + | + | + | + | + | + | - | + | + | + |
| 0,05 | + | + | + | + | + | + | + | + | + | + | + | + |
| 0,025 | + | + | + | + | + | + | + | + | + | + | + | + |
| 0,0125 | + | + | + | + | + | + | + | + | + | + | + | + |
| 0,00625 | + | + | + | + | + | + | + | + | + | + | + | + |

**TABLEAU C**

| (Composition conforme à l'invention : solution à 2 % → 0,001 % de Bardac® et 0,001 % de CHG) | | | | | | | | | | | | |
|---|---|---|---|---|---|---|---|---|---|---|---|---|
| Essai Souche | *S. aureus* ATCC 6538 | | | | *E. coli* ATCC 11229 | | | | *Candida albicans* ATCC 1031 | | | |
| Conc. % (vol/vol) | non | A | B | C | non | A | B | C | non | A | B | C |
| 2,0 | - | + | + | + | - | + | + | + | - | + | + | + |
| 1,0 | - | + | + | + | - | + | + | + | - | + | + | + |
| 0,5 | - | + | + | + | - | + | + | + | - | + | + | + |
| 0,1 | - | + | + | + | + | + | + | + | + | + | + | + |
| 0,05 | + | + | + | + | + | + | + | + | + | + | + | + |
| 0,025 | + | + | + | + | + | + | + | + | + | + | + | + |
| 0,0125 | + | + | + | + | + | + | + | + | + | + | + | + |
| 0,00625 | + | + | + | + | + | + | + | + | + | + | + | + |

Les tests dont les résultats sont illustrés aux Tableaux D, E et F ont été réalisés à une température de réaction de 22° avec une incubation des sous-cultures 72 heures à 37°C avec comme substances inactivantes : Tween® 80 3 % + saponine 3 % + cystéine 0,1 % + histidine 0,1 % ; ces Tableaux illustrent respectivement l'activité bactéricide et fongicide d'une solution aqueuse à 0,1 % de digluconate de chlorhexidine, d'une solution aqueuse à 0,1 % de Bardac 22® et d'un mélange à part égale des deux composés et montrent l'action des antiseptiques précités, en fonction du temps de contact, et font apparaître la même synergie d'action de l'association que ci-dessus, dans la mesure où l'association des deux composés permet de diminuer significativement leur concentration, pour l'obtention d'un même effet.

**TABLEAU D**

| (CHG seul) | | | | | | | | | | | | | | | | | | |
|---|---|---|---|---|---|---|---|---|---|---|---|---|---|---|---|---|---|---|
| Essai Souche | *S*. *aureus* ATCC 6538 organismes/ml : 8 x10⁸ | | | | | | *E*. *coli* ATCC 11229 organismes/ml : 2 x 10 | | | | | | *Candida albicans* ATCC 1031 organismes/ml : 8 x 10⁷ | | | | | |
| | Temps de désinfection (minutes) | | | | | | | | | | | | | | | | | |
| Conc. % (vol/vol) | 1 | 3 | 5 | 15 | 30 | 60 | 1 | 3 | 5 | 15 | 30 | 60 | 1 | 3 | 5 | 15 | 30 | 60 |
| 25 | + | + | + | + | - | - | + | + | + | - | - | - | + | + | + | - | - | - |
| 10 | + | + | + | + | + | - | + | + | + | - | - | - | + | + | + | + | - | - |
| 5 | + | + | + | + | + | + | + | + | + | + | - | - | + | + | + | + | - | - |
| 1 | + | + | + | + | + | + | + | + | + | + | + | + | + | + | + | + | + | - |
| 0,5 | + | + | + | + | + | + | + | + | + | + | + | + | + | + | + | + | + | + |
| 0,1 | + | + | + | + | + | + | + | + | + | + | + | + | + | + | + | + | + | + |
| 0,05 | + | + | + | + | + | + | + | + | + | + | + | + | + | + | + | + | + | + |

**TABLEAU E**

| (Bardac® seul) | | | | | | | | | | | | | | | | | | |
|---|---|---|---|---|---|---|---|---|---|---|---|---|---|---|---|---|---|---|
| Essai Souche | *S*. *aureus* ATCC 6538 organismes/ml : 8 x 10⁸ | | | | | | *E*. *coli* ATCC 11229 organismes/ml : 2 x 10⁹ | | | | | | *Candida albicans* ATCC 1031 organismes/ml : 8 x 10⁷ | | | | | |
| | Temps de désinfection (minutes) | | | | | | | | | | | | | | | | | |
| Conc. % (vol/vol) | 1 | 3 | 5 | 15 | 30 | 60 | 1 | 3 | 5 | 15 | 30 | 60 | 1 | 3 | 5 | 15 | 30 | 60 |
| 25 | + | - | - | - | - | - | - | - | - | - | - | - | - | - | - | - | - | - |
| 10 | + | - | - | - | - | - | + | + | - | - | - | - | - | - | - | - | - | - |
| 5 | + | + | + | - | - | - | + | + | + | - | - | - | + | + | - | - | - | - |
| 1 | + | + | + | + | - | - | + | + | + | + | + | + | + | + | + | + | - | - |
| 0,5 | + | + | + | + | + | + | + | + | + | + | + | + | + | + | + | + | + | + |
| 0,1 | + | + | + | + | + | + | + | + | + | + | + | + | + | + | + | + | + | + |
| 0,05 | + | + | + | + | + | + | + | + | + | + | + | + | + | + | + | + | + | + |

**TABLEAU F**

| (Composition conforme à l'invention) | | | | | | | | | | | | | | | | | | |
|---|---|---|---|---|---|---|---|---|---|---|---|---|---|---|---|---|---|---|
| Essai Souche | *S*. *aureus* ATCC 6538 organismes/ml : 8 x 10⁸ | | | | | | *E*. *coli* ATCC 11229 organismes/ml : 2 x 10⁹ | | | | | | *Candida albicans* ATCC 1031 organismes/ml : 8 x 10⁷ | | | | | |
| | Temps de désinfection (minutes) | | | | | | | | | | | | | | | | | |
| Conc. % (vol/vol) | 1 | 3 | 5 | 15 | 30 | 60 | 1 | 3 | 5 | 15 | 30 | 60 | 1 | 3 | 5 | 15 | 30 | 60 |
| 25 | + | - | - | - | - | - | - | - | - | - | - | - | + | - | - | - | - | - |
| 10 | + | + | + | - | - | - | - | - | - | - | - | - | + | - | - | - | - | - |
| 5 | + | + | + | + | - | - | + | - | - | - | - | - | + | + | + | - | - | - |
| 1 | + | + | + | + | + | + | + | + | + | + | - | - | + | + | + | + | + | + |
| 0,5 | + | + | + | + | + | + | + | + | + | + | + | + | + | + | + | + | + | + |
| 0,1 | + | + | + | + | + | + | + | + | + | + | + | + | + | + | + | + | + | + |
| 0,05 | + | + | + | + | + | + | + | + | + | + | + | + | + | + | + | + | + | + |

### EXAMPLE 5 : Action spermicide.

### 1. PROTOCOLE :

Les différents échantillons de sperme (une goutte de sperme/échantillon) sont obtenus à partir de volontaires sains.

### - Mesure de la vitalité :

Une goutte de sperme est traitée par l'éosinenigrosine. Un frottis est ensuite réalisé et cent spermatozoïdes sont examinés au microscope. Les cellules mortes sont colorées en rouge, soit totalement, soit partiellement. Les cellules vivantes sont incolores.

### - Substances étudiées :

Les substances étudiées ont été, le Bardac® et le digluconate de chlorhexidine, testés seuls et en association conformément à l'invention. Les différentes étapes du protocole sont indiquées aux Tableaux IV à VI.

Le temps de contact maximum des différentes substances et du sperme a été limité à 3 minutes afin de garantir l'efficacité de la méthode dans les conditions d'utilisation. La concentration des solutions actives dans le sperme a été limitée à 10 % afin de diminuer les risques de toxicité des produits pour les muqueuses.

**TABLEAU IV**

| | | **Temps de contact des substances et du sperme** | | | |
|---|---|---|---|---|---|
| **BARDAC** | | **1 Min** | | **3 Min** | |
| | | **Concentration des solutions dans le sperme** | | | |
| | | 5 % | 10 % | 5 % | 10 % |
| **Témoin à T0** | **Concentration des solutions en substances actives** | | | | |
| 82.8 ± 7.1 | 0,4 % | 10.7 ± 7.6 | 5 ± 6.4 | 0.8 ± 1.2 | 0.8 ± 1 |
| 81.4 ± 5.9 | 0,6 % | 10.5 ± 9.7 | 9.2 ± 12.9 | 2.2 ± 4.5 | 0.4 ± 0.9 |
| 81.4 ± 5.9 | 0,8 % | 9.7 ± 10.2 | 1.8 ± 2.5 | 0 | 0 |
| 79.7 + 5.3 | 1 % | 0 | 0 | 0 | 0 |

**TABLEAU V**

| | | **Temps de contact des substances et du sperme** | | | |
|---|---|---|---|---|---|
| **DIGLUCONATE DE CHLORHEXIDINE** | | **1 Min** | | **3 Min** | |
| | | **Concentration des solutions dans le sperme** | | | |
| | | 5 % | 10 % | 5 % | 10 % |
| **Témoin à T0** | **Concentration des solutions on substances actives** | | | | |
| 88 ± 6.2 % | 0,5 % | 77 ± 10.8 | 77.2 ± 5.6 | 78 ± 5.7 | 75,5 ± 4.8 |

**TABLEAU VI**

| | | **Temps de contact des substances et du sperme** | | | |
|---|---|---|---|---|---|
| **BARDAC + DIGLUCONATE DE CHLORHEXIDINE** | | **1 Min** | | **3 Min** | |
| | | **Concentration des solutions dans le sperme** | | | |
| | | 5 % | 10 % | 5 % | 10 % |
| **Témoin à T0** | **Concentration des solutions en substances actives** | | | | |
| 88.4 ± 5.4% | B 0.4 % + DC 0.5 % | 15.6 ± 13.2 | 5.1 ± 8 | 8.6 ± 10.8 | 2.4 ± 4.1 |
| | 0,8 | 21.3 ± 14 | 6.7 ± 3 | 18.3 ± 13.3 | 6.3 ± 7.8 |
| | 1 | 19 ± 11.1 | 10 ± 8.7 | 16 ± 9.8 | 9.3 ± 11.9 |
| | B 0.6 % + DC 0.5 % | 6.3 ± 11 | 4 ± 6.9 | 1.3 ± 2.3 | 1 ± 1.7 |
| | B 0.8 % + DC 0.5 % | 3.7 ± 6.3 | 0 | 2 ± 3.5 | 0 |

### EXEMPLE 6 : Etude du mélange Bardac 22®-digluconate de chlorhexidine en solution aqueuse.

Tous les mélanges ont été réalisés en pourcentage poids/poids dans des flacons plastiques placés à l'abri de la lumière à température ambiante (20°C +-1°C).

Une observation quotidienne de ces flacons a permis de détecter ou non la présence d'un précipité blanc. Celui-ci (quand il se forme) se trouve sur le culot du flacon en majorité, mais aussi sur les parois mouillées par le liquide.

### 1) Etude de solutions de Bardac®-chlorhexidine 50/50 à différentes concentrations :

L'étude a été faite dans un premier temps sur des solutions aqueuses à différentes concentrations en principes actifs, mais pour un mélange des deux principes actifs (Bardac® et digluconate de chlorhexidine) en proportion égale (50/50) :
Solution à 30 % de principes actifs Précipité à 24 h
Solution à 20 % de principes actifs Précipité à 24 h
Solution à 10 % de principes actifs Précipité à 24 h
Solution à 5 % de principes actifs Précipité à 24 h
Solution à 1 % de principes actifs Précipité à 24 h

### 2) Etude de solutions à 10 et 20 % de Bardac® et de chlorhexidine en différentes proportions :

Dans un deuxième temps, les concentrations en principes actifs ont été fixées à 10 % et 20 % mais on a fait varier les proportions des deux principes actifs.

**TABLEAU**

| RECAPITULATIF DES SOLUTIONS A 10 % DE PRINCIPES ACTIFS | | | |
|---|---|---|---|
| Rapport diglucon. chlorhex./Bardac® | % digluc. chlorhex. | % Bardac® | Observations |
| 0,5/99,5 | 0,05 | 9,95 | pas de précipité |
| 1/99 | 0,1 | 9,9 | pas de précipité |
| 2/98 | 0,2 | 9,8 | pas de précipité |
| 4/96 | 0,4 | 9,6 | pas de précipité |
| 8/92 | 0,8 | 9,2 | pas de précipité |
| 16/84 | 1,6 | 8,4 | pas de précipité |
| 25/75 | 2,5 | 7,5 | précipité à 2 mois |
| 35/65 | 3,5 | 6,5 | précipité à 9 jours |
| 40/60 | 4,0 | 6,0 | précipité à 6 jours |
| 50/50 | 5,0 | 5,0 | précipité à 24 h |

**TABLEAU**

| RECAPITULATIF DES SOLUTIONS A 20 % DE PRINCIPES ACTIFS | | | |
|---|---|---|---|
| Rapport diglucon. chlorhex./Bardac® | % digluc. chlorhex. | % Bardac® | Observations |
| 0,5/99,5 | 0,1 | 19,9 | pas de précipité |
| 1/99 | 0,2 | 19,8 | pas de précipité |
| 4/96 | 0,8 | 19,2 | pas de précipité |
| 2/98 | 0,4 | 19,6 | pas de précipité |
| 8/92 | 1,6 | 18,4 | pas de précipité |
| 16/84 | 3,2 | 16,8 | pas de précipité |
| 25/75 | 5,0 | 15,0 | précipité à 12 jours |
| 35/65 | 7,0 | 13,0 | précipité à 9 jours |
| 40/60 | 8,0 | 12,0 | précipité à 6 jours |
| 50/50 | 10,0 | 10,0 | précipité à 24 h |

### EXEMPLE 7 : Etude du mélange Bardac 22®-digluconate de chlorhexidine en solution alcoolique.

Les dissolutions dans les proportions indiquées pour l'eau sont identiques, lorsque les deux produits sont en solution dans du glycérol.

### EXEMPLE 8 : Etude de la capacité désinfectante, vis-à-vis des bactéries et des champignons, de microcapsules contenant une composition conforme à l'invention et incluses dans un tissu en polyvinyle, après piqûre dudit tissu par une aiguille.

### a) Matériel et méthode :

* Deux échantillons de microcapsules incluses dans des tissus en polyvinyle ou latex comprennent :
   - échantillon 1 : microcapsules contenant une composition conforme à l'invention, incluses dans du PVDC. L'épaisseur du tissu couvrant les microcapsules est d'environ 500 µm et l'épaisseur totale du tissu est d'environ 1 300 µm et la quantité de désinfectant disponible après déchirure du tissu par une aiguille (0,5x16 mm) est d'environ 1,5 µg ;
   - échantillon 2 : microcapsules contenant une composition conforme à l'invention de type paire I, incluses dans de l'EVA. L'épaisseur du tissu couvrant les microcapsules est d'environ 500 µm et la quantité de désinfectant disponible après déchirure du tissu par une aiguille (0,5x16 mm) est d'environ 0,4 µg.
* Les échantillons microbiologiques, pour effectuer le test, sont préparés en utilisant les microorganismes suivants : *Escherichia coli* ATCC 11229, *Staphylococcus aureus* ATCC 6538 et *Candida albicans* ATCC 10231.
   Les souches sont cultivées dans un milieu CSL (Merck) pendant 24 h à 37°C, puis des dilutions comprises entre 10⁻³ et 10⁻⁶ sont préparées dans une solution de chlorure de sodium à 0,9 %.
   1 ml de chacune des différentes cultures obtenues (diluée et non diluée) est additionné à 9 ml d'un pool de sérum humain inactivé. Le nombre de microorganismes par ml de la solution test est mentionné dans le Tableau VIII ci-après.
* Trois types d'aiguilles ont été utilisés : 0,7x30 mm (22 Gx1 1/4", Terumo) , 0,5x16 mm (25 Gx5/8", Terumo), 0,3x13 mm (30 Gx1/2", Microlance, Becton-Dickinson).

Ces aiguilles sont montées sur des seringues de 1 ml, trempées dans une solution microbienne telle que décrite ci-dessus (au moins 1 cm d'aiguille doit tremper dans la solution) et 0,1 ml de solution est aspiré. On perce ensuite les tissus ci-dessus décrits, placés dans des boîtes de Pétri recouvertes de Casoagar (Merck, milieu supplémenté avec 3 % de Tween® 80 et 0,3 % de lécithine), avec lesdites aiguilles.

Les emplacements où l'agar est inoculé par l'aiguille sont marqués, puis les milieux de culture sont incubés pendant 72 h à 37°C, avant la lecture des résultats.

### b) Résultats :

Le Tableau VII montre le nombre de cultures positives obtenues après perforation (5 perforations par essai), avec une aiguille 0,3x13 mm (30 Gx1/2", Microlance, Becton-Dickinson), et culture du tissu perforé sur milieu Casoagar (Merck) supplémenté comme précisé ci-dessus.

**TABLEAU VII**

| Solution microbienne | Echantillon 1 | | Echantillon 2 | |
|---|---|---|---|---|
| | Microcapsules | Contrôle | Microcapsules | Contrôle |
| *E*. *Coli* | | | | |
| 3.10⁸/ml | +---- | ++++- | +++-- | +++++ |
| 2.10⁵/ml | ----- | ----- | ----- | ----- |
| 10²/ml | ----- | ----- | ----- | ----- |

| *S*. *aureus* | | | | |
|---|---|---|---|---|
| 2.10⁸/ml | +++-- | +++++ | +++++ | +++++ |
| 2.10⁵/ml | ----- | ----- | ----- | ----- |
| 10²/ml | ----- | ----- | ----- | ----- |

| *C*. *albicans* | | | | |
|---|---|---|---|---|
| 3.10⁷/ml | ----- | +---- | ++--- | ++--- |
| 3.10⁵/ml | ----- | ----- | ----- | ----- |
| 10²/ml | ----- | ----- | ----- | ----- |
| + croissance de microorganismes, - absence de croissance | | | | |

Ces résultats montrent, en particulier, que des concentrations faibles de bactéries ou de champignons (10²/ml) sont absorbées ou éliminées mécaniquement par les tissus en polyvinyle ; une concentration de 10⁵ organismes/ml peut être considérée comme la limite ne produisant aucune croissance lorsque les plus petites aiguilles sont utilisées (0,3x13 mm, Tableau VIII) ; par contre, des résultats de même ordre sont obtenus avec les aiguilles de 0,7x30 mm et 0,5x16 mm, respectivement. On obtient une croissance uniquement pour les concentrations élevées en bactéries (10⁸/ml) ou en champignons (10⁷/ml), lors de la perforation des tissus contrôle uniquement (échantillon 1).

Ces résultats montrent également que les microcapsules de l'échantillon 1 (PVDC) sont plus efficaces que les microcapsules de l'échantillon 2 (EVA).

### c) Conclusion :

Les essais réalisés avec l'échantillon 1 montrent l'effet désinfectant quasi-instantané des compositions conformes à l'invention incorporées dans des microcapsules, dans des conditions proches de celles rencontrées à l'hôpital.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

## Revendications

1. Compositions à base d'antiseptiques, caractérisées en ce qu'elles sont constituées par une paire ammonium quaternaire-digluconate de chlorhexidine, dans laquelle chacun desdits antiseptiques est présent à des concentrations efficaces minimales diminuées d'au moins un facteur cinq par rapport aux concentrations efficaces minimales de chacun desdits antiseptiques pris isolément, en ce que ladite paire d'antiseptiques exerce une action rapide et polyvalente vis-à-vis des organismes suivants : bactéries à Gram positif, bactéries à Gram négatif, virus, champignons et spermatozoïdes, en ce que l'ammonium quaternaire est du chlorure de didécyldiméthylammonium, en ce que la concentration en chlorure de didécyldiméthylammonium est comprise entre 0,00005 et 0,4 % et la concentration en digluconate de chlorhexidine est comprise entre 0,00005 et 0,5 % et en ce que lesdites compositions sont susceptibles d'être incorporées dans des microsphérules ou microcapsules pouvant être incluses dans un matériau tel qu'un matériau en élastomère, lesquelles compositions n'étant libérées que lors d'une coupure, d'une piqûre ou d'une déchirure dudit matériau.

2. Compositions selon la revendication 1, caractérisées en ce qu'elles sont constituées par une paire ammonium quaternaire-digluconate de chlorhexidine dans laquelle les rapports chlorure de didécyldiméthylammonium:digluconate de chlorhexidine sont compris entre 99,5:0,5 et 84:16.

3. Compositions selon la revendication 1, caractérisées en ce qu'elles comprennent ces deux antiseptiques à des concentrations identiques, de préférence entre 0,00005 et 0,04 %.

4. Compositions selon l'une quelconque des revendications 1 à 3, caractérisées en ce qu'elles sont associées à au moins un agent de dissolution choisi dans le groupe constitué par l'eau et les polyols.

5. Compositions selon la revendication 4, caractérisées en ce que lesdits polyols sont choisis parmi le polypropylèneglycol, le polyéthylèneglycol ou le glycérol.

6. Utilisation des compositions selon l'une quelconque des revendications 1 à 5, pour l'obtention d'un médicament polyvalent à action rapide bactéricide, fongicide et virucide.

7. Utilisation d'une composition selon l'une quelconque des revendications 1 à 5, à la préparation de microsphérules ou microcapsules susceptibles d'être incluses dans un matériau en élastomère, une bande de tissu ou un pansement.

## Claims

1. Antiseptics-based compositions characterised in that they are constituted by a quaternary ammonium-chlorohexidine digluconate pairing in which each of the said antiseptics is present at minimal effective concentrations diminished by at least a factor of five in relation to the minimal effective concentrations of each of the said antiseptics taken in isolation, in that the said antiseptic pair exerts a rapid and polyvalent action on the following organisms: gram positive bacteria, gram negative bacteria, viruses, fungus and spetmatozoids, in that the quaternary ammonium is didecyl-dimethyl ammonium chloride, in that the concentration of didecyl dimethyl ammonium chloride is comprised between 0.00005 and 0.4% and the concentration of chlorohexidine digluconate is comprised between 0.00005 and 0.5% and in that the said compositions are capable of being incorporated into microcapsules or microspheres adapted for inclusion in a material such as an elastomeric material, the said compositions being released only upon a cutting, piercing or tearing of the said material.

2. Compositions according to claim 1, characterised in that they are constituted by a quaternary ammonium-chlorohexidine digluconate pairing in which the ratios of didecyl dimethyl ammonium chloride: chlorohexidine digluconate are comprised between 99.5 : 0.5 and 84 : 16.

3. Compositions according to claim 1, characterised in that they comprise these two antiseptics as identical concentrations, preferably between 0.00005 and 0.04%.

4. Compositions according to any one of claims 1 to 3, characterised in that they are associated with at least one dissolution agent chosen from the group constituted by water and the polyols.

5. Compositions according to claim 4, characterised in that the said polyols are chosen from polypropylene glycol, polyethylene glycol or glycerol.

6. Use of the compositions according to any one of claims 1 to 5 for obtaining a polyvalent medicament of rapid bactericidal, fungicidal and virocidal action.

7. Use of a composition according to any one of claims 1 to 5 in the preparation of microspheres or microcapsules adapted to be included in an elastomeric material, a strip of cloth or a dressing.

## Patentansprüche

1. Zusammensetzung auf der Basis von Antiseptika, dadurch **gekennzeichnet,** daß sie aus einem Paar quarternäres Ammoniumsalz-Chlorhexidindigluconat bestehen, worin jedes der Antiseptika in minimalen, wirksamen Konzentrationen vorhanden ist, die um mindestens einen Faktor von fünf vermindert sind, bezogen auf die minimalen, wirksamen Konzentrationen jedes der Antiseptika isoliert genommen, daß das Paar von Antiseptika eine rasche und polyvalente Wirkung gegenüber den folgenden Organismen ausübt: grampositive Bakterien, gramnegative Bakterien, Viren, Pilze und Spermazotoide, daß das quarternäre Ammoniumsalz Didecyldimethylammoniumchlorid ist, daß die Konzentration von Didecyldimethylammoniumchlorid zwischen 0,00005 und 0,4% liegt und die Konzentration an Chlorhexidindigluconat zwischen 0,00005 und 0,5% liegt und daß die Zusammensetzungen in Mikrokügelchen oder Mikrokapseln eingearbeitet werden können, die in einem Material, wie ein Elastomerenmaterial, eingeschlossen werden können, wobei die Zusammensetzungen nur durch Anschneiden, Anstechen oder Einreißen des Materials freigesetzt werden.

2. Zusammensetzungen nach Anspruch 1, dadurch **gekennzeichnet,** daß sie aus einem Paar quarternäres Ammoniumsalz-Chlorhexidindigluconat bestehen, worin die Verhältnisse Didecyldimethylammoniumchlorid:Chlorhexidindigluconat zwischen 99,5:0,5 und 84:16 liegen.

3. Zusammensetzungen nach Anspruch 1, dadurch **gekennzeichnet,** daß sie diese zwei Antiseptika in identischen Konzentration, bevorzugt zwischen 0,00005 und 0,04%, umfassen.

4. Zusammensetzungen nach einem der Ansprüche 1 bis 3, dadurch **gekennzeichnet,** daß sie mit mindestens einem Lösungsmittel, ausgewählt aus der Gruppe bestehend aus Wasser und Polyolen, assoziiert sind.

5. Zusammensetzungen nach Anspruch 4, dadurch **gekennzeichnet,** daß die Polyole aus Polypropylenglykol, Polyethylenglykol oder Glycerin ausgewählt sind.

6. Verwendung der Zusammensetzungen nach einem der Ansprüche 1 bis 5 zum Erhalt eines polyvalenten Medikaments mit rascher bakterizider, fungizider und viruzider Wirkung.

7. Verwendung einer Zusammensetzung nach einem der Ansprüche 1 bis 5 zur Herstellung von Mikrokügelchen oder Mikrokapseln, die in einem elastomeren Material, einem Gewebeband oder einem Verband eingeschlossen werden können.
